# EUROPEAN PATENT APPLICATION

(11) **EP 4 289 816 A1**
(43) Date of publication of application: **13.12.2023**
(21) Application number: 21951006.2
(22) Date of filing: 21.07.2021
(51) Int. Cl.: C07C 253/02, C07C 255/51

(54) **METHOD FOR PREPARING PHTHALONITRILE-BASED COMPOUND**

(71) Applicant: Korea Kumho Petrochemical Co., Ltd., Seoul 04542 (KR)
(72) Inventor: ROH, Kee Yoon, Daejeon 34021 (KR); CHO, Nam Hyun, Daejeon 34304 (KR); KIM, Dong Eon, Daejeon 34052 (KR)
(74) Representative: Beck & Rössig European Patent Attorneys
(86) International application number: PCT/KR2021/009452
(87) International publication number: WO 2023/003058

(57) **Abstract**

One embodiment provides a method of preparing a phthalonitrile-based compound, including: (a) preparing a mixture including a phthalic acid-based compound and a nitrile-based compound; and (b) reacting the mixture, wherein step (b) is performed under supercritical conditions of the nitrile-based compound.

## Description

### BACKGROUND

### 1. Field of the Invention

The present invention relates to a method of preparing a phthalonitrile-based compound, and more particularly, to a method of preparing a phthalonitrile-based compound directly from a phthalic acid-based compound.

### 2. Discussion of Related Art

Phthalonitrile-based compounds are important compounds used as intermediates in the preparation of fiber-forming linear polymers. In addition, phthalonitrile-based compounds are used as organic synthesis intermediates for various fine chemical products such as amines, acid-amides, and complex nitrogen dyes, and it is also a high value-added raw material used in plasticizers, alkyd resin modifiers, pesticides, etc.

Conventionally, a phthalonitrile-based compound was prepared by contacting and dehydrating a xylene compound with ammonia and an oxygen-containing gas in the presence of an oxidation catalyst. However, since this method uses ammonia gas, which is a harmful chemical, and is an ammoxidation reaction performed in the presence of a catalyst at high temperature and high pressure, the process is complicated, and high-boiling-point impurities must be purified and separated through distillation, so there is a problem of difficulty in removing by-products. In addition, in this conventional method of preparing a phthalonitrile-based compound, the yield of the product varies depending on the type of catalyst used in the ammoxidation reaction and the oxygen-containing gas ratio, and the conversion rate of the xylene compound as a precursor fluctuates with the reaction temperature, making it difficult to control the process.

Accordingly, there is an increasing demand for a process of preparing a high-purity phthalonitrile-based compound in an economical and environmentally friendly manner.

### SUMMARY OF THE INVENTION

One object of this specification is to provide a method of directly preparing a phthalonitrile-based compound from a phthalic acid-based compound.

In addition, another object of this specification is to provide a method of preparing an environmentally friendly phthalonitrile-based compound.

According to one aspect, the present invention provides a method of preparing a phthalonitrile-based compound, including: (a) preparing a mixture including a phthalic acid-based compound and a nitrile-based compound; and (b) reacting the mixture, wherein step (b) is performed under supercritical conditions of the nitrile-based compound.

In one embodiment, the phthalic acid-based compound may be isophthalic acid, terephthalic acid, or a mixture thereof.

In one embodiment, the nitrile-based compound may be at least one selected from the group consisting of hydrogen cyanide, acetonitrile, acrylonitrile, butyronitrile, isobutyronitrile, pivalonitrile, succinonitrile, fumaronitrile, crotonitrile, and benzonitrile.

In one embodiment, the nitrile-based compound may be acetonitrile.

In one embodiment, the mixture of step (a) may be comprised of a phthalic acid-based compound and a nitrile-based compound.

In one embodiment, in step (a), the content of the nitrile-based compound may be 1 to 500 parts by weight based on 1 part by weight of the phthalic acid-based compound.

In one embodiment, in step (a), the water content of the mixture may be less than 6,000 ppm.

In one embodiment, step (b) may be performed under conditions of 260 to 350 °C and 40 to 200 bar.

In one embodiment, step (b) may be performed for 1 to 500 minutes.

In one embodiment, after step (b), (c) separating the product of the step (b) may be further included.

In one embodiment, the residual compound separated in step (c) may be reused in step (a).

### DETAILED DESCRIPTION OF EXEMPLARY EMBODIMENTS

Hereinafter, one aspect of this specification will be described with reference to an embodiment. However, the description of this specification may be implemented in several different forms, and thus is not limited to the embodiments described herein. In addition, in order to clearly explain each content for the implementation of this specification, parts not related to the description or widely known in the art have been omitted.

Throughout the specification, when a part is "connected" to another part, this includes not only the case where it is "directly connected" but also the case where it is "indirectly connected" with another member interposed therebetween. In addition, when a part is said to "include" a component, this means that other components may be further included, not excluded, unless specifically stated otherwise.

When a range of numerical values is recited herein, the values have the precision of the significant figures provided in accordance with the standard rules in chemistry for significant figures, unless a specific range is otherwise stated. For example, 10 includes the range of 5.0 to 14.9, and 10.0 includes the range of 9.50 to 10.49. In addition, when examples of a plurality of numerical values are described in this specification, these examples may include the intervening values unless explicitly stated to exclude these intervening values. For example, "x₁, x₂, x₃ or x₄" may include the ranges of "x₁ to x₂," "x₁ to x₃," "x₁ to x₄," "x₂ to x_{3,}" "x₂ to x₄," or "x₃ to x₄."

In this specification, "supercritical condition" means a state that satisfies a condition equal to or higher than a critical point, which is an end point of a phase equilibrium curve. For example, when a critical temperature of compound A is T_{c} and a critical pressure is P_{c}, a supercritical condition of compound A means a state in which the temperature is T_{c} or more and the pressure is P_{c} or more.

Hereinafter, embodiments of this specification will be described in detail.

### Method of preparing phthalonitrile-based compound

A method of preparing a phthalonitrile-based compound according to one aspect includes: (a) preparing a mixture including a phthalic acid-based compound and a nitrile-based compound; and (b) reacting the mixture, wherein step (b) is performed under supercritical conditions of the nitrile-based compound.

The phthalic acid-based compound may be a compound having an aromatic ring and two or more carboxyl groups. In one embodiment, the phthalic acid-based compound may be isophthalic acid, terephthalic acid, or a mixture thereof.

The nitrile-based compound may be at least one selected from the group consisting of hydrogen cyanide, acetonitrile, acrylonitrile, butyronitrile, isobutyronitrile, pivalonitrile, succinonitrile, fumaronitrile, crotonitrile, and benzonitrile, but is not limited thereto. For example, when the nitrile-based compound is hydrogen cyanide, step (b) may be performed at 183.5 °C or higher and 50 bar or higher. When the nitrile-based compound is acetonitrile, step (b) may be performed at 272 °C or higher and 48.7 bar or higher. When the nitrile-based compound is acrylonitrile, step (b) may be performed at 267 °C or higher and 46 bar or higher. When the nitrile-based compound is butyronitrile, step (b) may be performed at 309 °C or higher and 37.8 bar or higher. When the nitrile-based compound is isobutyronitrile, step (b) may be performed at 336 °C or higher and 40 bar or higher. When the nitrile-based compound is pivalonitrile, step (b) may be performed at 343 °C or higher and 34.4 bar or higher. In addition, the conditions of step (b) may be changed according to the type of nitrile-based compound. Accordingly, all of the above conditions are exemplary and do not limit the scope of this specification. The nitrile-based compound may be both a solvent and a reactant.

In step (b), the mixture may be reacted without additional additives such as ammonia, high concentration oxygen, or a catalyst. Since the reaction of step (b) may be performed without a separate additive, the mixture of step (a) may be comprised of a phthalic acid-based compound and a nitrile-based compound, but is not limited thereto. For example, the mixture may be obtained by dissolving a solid phthalic acid-based compound in a nitrile-based compound solvent, but is not limited thereto.

In step (a), the content of the nitrile-based compound may be 1 to 500 parts by weight based on 1 part by weight of the phthalic acid-based compound. For example, the content of the nitrile-based compound may be 1 part by weight, 5 parts by weight, 10 parts by weight, 15 parts by weight, 20 parts by weight, 25 parts by weight, 30 parts by weight, 35 parts by weight, 40 parts by weight, 45 parts by weight, 50 parts by weight, 55 parts by weight, 60 parts by weight, 65 parts by weight, 70 parts by weight, 75 parts by weight, 80 parts by weight, 85 parts by weight, 90 parts by weight, 95 parts by weight, 100 parts by weight, 105 parts by weight, 110 parts by weight, 115 parts by weight, 120 parts by weight, 125 parts by weight, 130 parts by weight, 135 parts by weight, 140 parts by weight, 145 parts by weight, 150 parts by weight, 155 parts by weight, 160 parts by weight, 165 parts by weight, 170 parts by weight, 175 parts by weight, 180 parts by weight, 185 parts by weight, 190 parts by weight, 195 parts by weight, 200 parts by weight, 205 parts by weight, 210 parts by weight, 215 parts by weight, 220 parts by weight, 225 parts by weight, 230 parts by weight, 235 parts by weight, 240 parts by weight, 245 parts by weight, 250 parts by weight, 255 parts by weight, 260 parts by weight, 265 parts by weight, 270 parts by weight, 275 parts by weight, 280 parts by weight, 285 parts by weight, 290 parts by weight, 295 parts by weight, 300 parts by weight, 305 parts by weight, 310 parts by weight, 315 parts by weight, 320 parts by weight, 325 parts by weight, 330 parts by weight, 335 parts by weight, 340 parts by weight, 345 parts by weight, 350 parts by weight, 355 parts by weight, 360 parts by weight, 365 parts by weight, 370 parts by weight, 375 parts by weight, 380 parts by weight, 385 parts by weight, 390 parts by weight, 395 parts by weight, 400 parts by weight, 405 parts by weight, 410 parts by weight, 415 parts by weight, 420 parts by weight, 425 parts by weight, 430 parts by weight, 435 parts by weight, 440 parts by weight, 445 parts by weight, 450 parts by weight, 455 parts by weight, 460 parts by weight, 465 parts by weight, 470 parts by weight, 475 parts by weight, 480 parts by weight, 485 parts by weight, 490 parts by weight, 495 parts by weight, or 500 parts by weight, based on 1 part by weight of the phthalic acid-based compound, including intermediate ranges thereof. As the content of the nitrile-based compound increases compared to the content of the phthalic acid-based compound, although the purity of the product may increase, an excessive amount of the nitrile-based compound may be economically disadvantageous.

In one embodiment, in step (a), the water content of the mixture may be less than 6,000 ppm. For example, the water content of the mixture may be less than 6,000 ppm, less than 5,000 ppm, less than 4,000 ppm, less than 3,000 ppm, less than 2,000 ppm, less than 1,000 ppm, less than 750 ppm, less than 500 ppm, or less than 250 ppm. The lower the water content of the mixture, the higher the purity of the product.

The reaction of step (b) may be di-nitrilation through a direct substitution reaction between a carboxyl group and a nitrile group, and an example thereof may be expressed as the following reaction scheme.

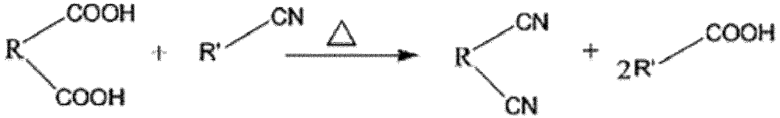

In the reaction scheme, R is an aromatic ring such as phenylene, and R' may be an alkyl group having 1 to 20 carbon atoms, for example, a methyl group, an ethyl group, an isopropyl group, a t-butyl group, etc.

The reaction may be performed when the temperature and pressure conditions in step (b) are above the critical point of the nitrile-based compound. Step (b) may be performed under conditions of 260 to 350 °C and 40 to 200 bar. For example, in step (b), the reaction temperature may be 260 °C, 265 °C, 270 °C, 275 °C, 280 °C, 285 °C, 290 °C, 295 °C, 300 °C, 305 °C, 310 °C, 315 °C, 320 °C, 325 °C, 330 °C, 335 °C, 340 °C, 345 °C, or 350 °C. For example, in step (b), the reaction pressure may be 40 bar, 45 bar, 50 bar, 55 bar, 60 bar, 65 bar, 70 bar, 75 bar, 80 bar, 85 bar, 90 bar, 95 bar, 100 bar, 105 bar, 110 bar, 115 bar, 120 bar, 125 bar, 130 bar, 135 bar, 140 bar, 145 bar, 150 bar, 155 bar, 160 bar, 165 bar, 170 bar, 175 bar, 180 bar, 185 bar, 190 bar, 195 bar, or 200 bar. When the reaction temperature in step (b) is excessively low, the purity of the product may decrease or the reaction may not be carried out, and when the reaction temperature is excessively high, the generation of by-products may increase and the purity may decrease. When the reaction pressure in step (b) is excessively low, the reaction may not be carried out, and when the reaction pressure is excessively high, safety may be compromised.

Step (b) may be performed for 1 to 500 minutes. For example, step (b) may be performed for 1 minute, 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes, 240 minutes, 245 minutes, 250 minutes, 255 minutes, 260 minutes, 265 minutes, 270 minutes, 275 minutes, 280 minutes, 285 minutes, 290 minutes, 295 minutes, 300 minutes, 305 minutes, 310 minutes, 315 minutes, 320 minutes, 325 minutes, 330 minutes, 335 minutes, 340 minutes, 345 minutes, 350 minutes, 355 minutes, 360 minutes, 365 minutes, 370 minutes, 375 minutes, 380 minutes, 385 minutes, 390 minutes, 395 minutes, 400 minutes, 405 minutes, 410 minutes, 415 minutes, 420 minutes, 425 minutes, 430 minutes, 435 minutes, 440 minutes, 445 minutes, 450 minutes, 455 minutes, 460 minutes, 465 minutes, 470 minutes, 475 minutes, 480 minutes, 485 minutes, 490 minutes, 495 minutes, or 500 minutes, including intermediate ranges thereof. As the reaction time in step (b) increases, although the purity of the product may increase, when the reaction time is excessively long, productivity may decrease.

After step (b), (c) separating the product of step (b) may be further included. The separation in step (c) may be separation into a phthalonitrile-based compound and a residual compound, and may be performed according to various known methods such as distillation. The residual compound may include, for example, at least one selected from the group consisting of unreacted phthalic acid-based compounds, unreacted nitrile-based compounds, and phthalic acid-nitrile-based compounds in which only a portion of the carboxyl groups of the phthalic acid-based compounds have reacted, but is not limited thereto.

The residual compound separated in step (c) may be reused in step (a). The method of preparing a phthalonitrile-based compound according to an embodiment of the present specification can be performed without the use of a separate additive such as a catalyst, so that the residual compound can be reused without separate purification.

In the method of preparing a phthalonitrile-based compound according to one aspect, the purity of the product may be 60% or more, 65% or more, 70% or more, 75% or more, 80% or more, or 85% or more.

Hereinafter, examples of this specification will be described in more detail. However, the following experimental results describe only representative experimental results among the examples, and the scope and content of the present invention may not be construed as being reduced or limited by the examples. Each effect of the various embodiments of the present invention not explicitly presented below will be specifically described in the corresponding section.

### Example 1

5 parts by weight of isophthalic acid (IPA) and 100 parts by weight of acetonitrile (ACN) were input to a 1,000 mL reactor (autoclave) equipped with a stirrer to form a reaction system. Nitrogen was substituted three times at a pressure of 2 to 3 bar inside the reactor. While stirring at 400 rpm under atmospheric pressure, the internal temperature of the reactor was raised to 280 to 300 °C. The reaction was performed for 4 hours while maintaining the reaction temperature, and the reaction pressure was 70 to 100 bar. After the reaction was completed, the reaction system was cooled to room temperature. Thereafter, the reaction system was distilled under reduced pressure to separate acetonitrile and isophthalonitrile (IPN). The acetonitrile was reused, and the isophthalonitrile was analyzed by gas chromatography (GC) to confirm the purity of the product.

### Example 2

5 parts by weight of isophthalic acid and 100 parts by weight of acetonitrile were input to a 1,000 mL reactor equipped with a stirrer to form a reaction system. Nitrogen was substituted three times at a pressure of 2 to 3 bar inside the reactor. While stirring at 400 rpm under atmospheric pressure, the internal temperature of the reactor was raised to 290 °C. The reaction was performed for 1 to 6 hours while maintaining the reaction temperature, and the reaction pressure was 90 to 95 bar. After the reaction was completed, the reaction system was cooled to room temperature. Thereafter, the reaction system was distilled under reduced pressure to separate acetonitrile and isophthalonitrile. The acetonitrile was reused, and the isophthalonitrile was analyzed by gas chromatography to confirm the purity of the product.

### Example 3

5 parts by weight of isophthalic acid and 50 to 150 parts by weight of acetonitrile were input to a 1,000 mL reactor equipped with a stirrer to form a reaction system. Nitrogen was substituted three times at a pressure of 2 to 3 bar inside the reactor. While stirring at 400 rpm under atmospheric pressure, the internal temperature of the reactor was raised to 290 °C. The reaction was performed for 4 hours while maintaining the reaction temperature, and the reaction pressure was 90 to 95 bar. After the reaction was completed, the reaction system was cooled to room temperature. Thereafter, the reaction system was distilled under reduced pressure to separate acetonitrile and isophthalonitrile. The acetonitrile was reused, and the isophthalonitrile was analyzed by gas chromatography to confirm the purity of the product.

### Example 4

5 parts by weight of isophthalic acid and 100 parts by weight of acetonitrile were input to a 1,000 mL reactor equipped with a stirrer to form a reaction system. Nitrogen was substituted three times at a pressure of 2 to 3 bar inside the reactor. A water content of the reaction system was adjusted to 260 to 6,000 ppm. While stirring at 400 rpm under atmospheric pressure, the internal temperature of the reactor was raised to 280 °C. The reaction was performed for 4 hours while maintaining the reaction temperature, and the reaction pressure was 70 to 75 bar. After the reaction was completed, the reaction system was cooled to room temperature. Thereafter, the reaction system was distilled under reduced pressure to separate acetonitrile and isophthalonitrile. The acetonitrile was reused, and the isophthalonitrile was analyzed by gas chromatography to confirm the purity of the product.

### Example 5

5 parts by weight of terephthalic acid (TPA) and 100 parts by weight of acetonitrile were input to a 1,000 mL reactor equipped with a stirrer to form a reaction system. Nitrogen was substituted three times at a pressure of 2 to 3 bar inside the reactor. While stirring at 400 rpm under atmospheric pressure, the internal temperature of the reactor was raised to 280 to 290 °C. The reaction was performed for 4 hours while maintaining the reaction temperature, and the reaction pressure was 75 to 95 bar. After the reaction was completed, the reaction system was cooled to room temperature. Thereafter, the reaction system was distilled under reduced pressure to separate acetonitrile and terephthalonitrile (TPN). The acetonitrile was reused, and the terephthalonitrile was analyzed by gas chromatography to confirm the purity of the product.

The reaction conditions and product purities of Examples 1 to 5 are summarized in Table 1 below.

**[Table 1]**

| Classificat ion | IPA (parts by weig ht) | TPA (parts by weig ht) | ACN (parts by weig ht) | Stirri ng speed (rpm) | Reaction temperat ure (°C) | Reacti on pressu re (bar) | Reacti on time (hr) | Wate r conte nt (ppm ) | Puri ty (%) |
|---|---|---|---|---|---|---|---|---|---|
| Example 1-1 | 5 | 0 | 100 | 400 | 280 | 70-75 | 4 | - | 76.3 |
| Example 1-2 | 5 | 0 | 100 | 400 | 290 | 90-95 | 4 | - | 80.5 |
| Example 1-3 | 5 | 0 | 100 | 400 | 300 | 95-100 | 4 | - | 79.7 |
| Example 2-1 | 5 | 0 | 100 | 400 | 290 | 90-95 | 1 | - | 68.0 |
| Example 2-2 | 5 | 0 | 100 | 400 | 290 | 90-95 | 4 | - | 80.5 |
| Example 2-3 | 5 | 0 | 100 | 400 | 290 | 90-95 | 6 | - | 81.8 |
| Example 3-1 | 5 | 0 | 50 | 400 | 290 | 90-95 | 4 | - | 70.8 |
| Example 3-2 | 5 | 0 | 100 | 400 | 290 | 90-95 | 4 | - | 80.5 |
| Example 3-3 | 5 | 0 | 150 | 400 | 290 | 90-95 | 4 | - | 79.9 |
| Example 4-1 | 5 | 0 | 100 | 400 | 280 | 70-75 | 4 | 260 | 79.2 |
| Example 4-2 | 5 | 0 | 100 | 400 | 280 | 70-75 | 4 | 400 | 75.2 |
| Example 4-3 | 5 | 0 | 100 | 400 | 280 | 70-75 | 4 | 6,000 | 57.8 |
| Example 5-1 | 0 | 5 | 100 | 400 | 280 | 75-80 | 4 | - | 82.5 |
| Example 5-2 | 0 | 5 | 100 | 400 | 290 | 90-95 | 4 | - | 86.9 |

Referring to Example 1, when the reaction temperature was increased, the purity of isophthalonitrile, which is the product, increased, but when the reaction temperature was excessively high, by-products increased and the purity decreased.

Referring to Example 2, as the reaction time increased, the purity of isophthalonitrile, which is the product, increased.

Referring to Example 3, it was confirmed that the purity of isophthalonitrile, which is the product, increased as the amount of isophthalic acid compared to acetonitrile decreased.

Referring to Example 4, as the water content in the reaction system increased, the by-products increased and the purity of isophthalonitrile, which is the product, decreased.

Referring to Example 5, it can be confirmed that terephthalonitrile may be prepared by a similar reaction mechanism using terephthalic acid instead of isophthalic acid. In addition, as the reaction temperature increased, the purity of terephthalonitrile, which is the product, increased.

Referring to Examples 1 to 5, unlike the conventional preparation process using an ammoxidation reaction using harmful compounds such as ammonia and acid catalysts, in one embodiment of the present invention, a phthalonitrile-based compound may be prepared in high yield from a solid phthalic acid-based compound without a separate catalyst or additive input.

Specifically, in Examples 1 to 5, isophthalic acid or terephthalic acid, which is a phthalic acid-based compound, was used as a reactant, and acetonitrile, which is an organic nitrile, was used as a solvent and a reactant. The mixture of these compounds is heated directly without a separate catalyst or additive to form a high-temperature, highpressure supercritical state (T_{c}: 275 °C or higher, P_{c}: 48 bar or higher) and induce an exchange reaction between the acid and nitrile to directly generate a phthalonitrile-based compound.

### Advantageous Effects

According to one aspect, it is possible to directly prepare a phthalonitrile-based compound from a phthalic acid-based compound.

According to another aspect, the phthalonitrile-based compound can be prepared in an environmentally friendly manner.

The effects described in this specification are not limited to the above-described effects, and it should be understood to include all effects that can be inferred from the configurations described in the detailed description or claims of this specification.

The description of this specification described above is for illustrative purposes, and it should be understood that those of ordinary skill in the art to which one aspect of this specification belongs can easily modify it into other specific forms without changing the technical spirit or essential features described in this specification. Therefore, it should be understood that the embodiments described above are illustrative in all respects and not restrictive. For example, each component described as a single type may be implemented in a distributed form, and likewise, components described as distributed may be implemented in a combined form.

The scope of this specification is indicated by the following claims, and all changes or modifications derived from the meaning and scope of the claims and their equivalents should be construed as being included in the scope of this specification.

## Claims

1. A method of preparing a phthalonitrile-based compound, comprising:
(a) preparing a mixture including a phthalic acid-based compound and a nitrile-based compound; and
(b) reacting the mixture,
wherein step (b) is performed under supercritical conditions of the nitrile-based compound.

2. The method of claim 1, wherein the phthalic acid-based compound is isophthalic acid, terephthalic acid, or a mixture thereof.

3. The method of claim 1, wherein the nitrile-based compound is one or more selected from the group consisting of hydrogen cyanide, acetonitrile, acrylonitrile, butyronitrile, isobutyronitrile, pivalonitrile, succinonitrile, fumaronitrile, crotonitrile, and benzonitrile.

4. The method of claim 3, wherein the nitrile-based compound is acetonitrile.

5. The method of claim 1, wherein the mixture of step (a) is comprised of the phthalic acid-based compound and the nitrile-based compound.

6. The method of claim 1, wherein in step (a), a content of the nitrile-based compound is 1 to 500 parts by weight based on 1 part by weight of the phthalic acid-based compound.

7. The method of claim 1, wherein in step (a), a water content of the mixture is less than 6,000 ppm.

8. The method of claim 1, wherein step (b) is performed under conditions of 260 to 350 °C and 40 to 200 bar.

9. The method of claim 1, wherein step (b) is performed for 1 to 500 minutes.

10. The method of claim 1, further comprising (c) separating the product of step (b) after step (b).

11. The method of claim 10, wherein a residual compound separated in step (c) is reused in step (a).
